# EUROPEAN PATENT SPECIFICATION

(11) **EP 0 375 350 B1**
(45) Date of publication and mention of the grant of the patent: **20.04.1994**
(21) Application number: 89313263.9
(22) Date of filing: 19.12.1989
(51) Int. Cl.: A61K 31/785, C08F 8/44

(54) **Cross-linked vinylpyridinium polymers**
Vinylpyridinium vernetztes Polymer
Polyméres rèticulès de vinylpiridinium

(30) Priority: 21.12.1988 GB 8829835
(43) Date of publication of application: 27.06.1990
(73) Proprietor: SMITH KLINE & FRENCH LABORATORIES LIMITED, Welwyn Garden City Hertfordshire, AL7 1EY (GB)
(72) Inventor: Jaxa-Chamiec, Albert Andrzej, Welwyn Hertfordshire AL6 9AR (GB); Cooper, David Gwyn, Welwyn Hertfordshire AL6 9AR (GB)
(74) Representative: Giddings, Peter John, Dr.

(56) References cited:
- EP-A- 0 162 388
- DE-A- 2 556 082
- GB-A- 2 093 848
- US-A- 2 860 096
- US-A- 3 787 474

## Description

The present invention relates to novel polyvinylpyridinium anion exchange resins, processes for their preparation, pharmaceutical compositions containing them and their use in the lowering of plasma cholesterol levels in humans.

Coronary Heart Disease (CHD) is one of the most serious health problems of contemporary society. Worldwide epidemiological studies have shown that the incidence of CHD is related to a number of independent risk factors, in particular, for example, high concentrations of serum cholesterol (hypercholesterolaemia). Such adverse factors lead to atherosclerosis, and ultimately, in severe cases, intermittent claudication, cerebrovascular insufficiency, thrombosis and cardiac arrest.

It is known that ion exchange resins, in particular polystyrene resins can be used as sequestering agents to bind non-absorbed bile acids and salts in the intestinal tract, forming complexes which are then excreted in the faeces. This sequestering leads to a decrease in the amount of bile acids returning to the liver via enterohepatic circulation. The synthesis of replacement bile acids from hepatic cholesterol depletes hepatic cholesterol, regulates hepatic LDL receptors and consequently reduces plasma cholesterol levels. Such sequestering resins have been recognised as useful for the treatment of hypercholesterolaemia. In addition, it is now proven that reducing serum cholesterol with bile acid sequestrants has a beneficial effect on protecting against the occurrence of coronary heart disease.

One particular agent which is currently used to lower serum cholesterol levels in humans by binding bile acids in the intestinal tract is cholestyramine. Cholestyramine is a cross-linked anion exchange polystyrene resin bearing an ionised trimethylammonium group bound to the polymer backbone. However, the use of this agent is associated with a number of undesirable side-effects, for example, it is unpalatable and must be taken in high doses and causes, in some cases, bloating, constipation and other gut side-effects, and its ability to bind bile acids is inefficient with respect to the amounts of resin which it is necessary to use. There therefore remains a need for the provision of alternative agents in this area of therapy.

Vinylpyridinium polymers are known in the art. For example DT 2556082 discloses certain non-cross linked pyridine oligomers which are said to have anti-coagulant activity. These polymers are water-soluble and are not disclosed as having any bile-acid sequestering activity. US 4798870 discloses further non-cross linked vinylpyridinium polymers and their use as decontaminating agents, but again no bile acid sequestering activity is noted for the polymers; and J 1052728-A discloses further such non-cross linked polymers which are said to be dermal absorption accelerators.

In addition to the foregoing, certain prior art disclosures indicate that vinylpyridinium polymers also have bile acid sequestering activity. In particular, FR 70.33007 discloses a series of linear vinylpyridinium polymers; and US 3787474 discloses a related series of cross-linked vinylpyridinium polymers.
In both cases however there are no specific disclosures of such polymers or details of their levels of sequestering activity and the general disclosure of each document is limited only to pyridinium polymers in which the nitrogen atom in the pyridine ring bears a methyl, ethyl or propyl group.

It has now been found that certain water-insoluble cross-linked vinylpyridinium polymers in particular those bearing a long-chain alkyl group comprising at least 6 carbon atoms polymers exhibit potent properties as bile acid sequestrants in animal models and are expected to be of use in therapy in the treatment of, for example, atherosclerosis.

The present invention provides in a first aspect, cross-linked polymers of structure (I): 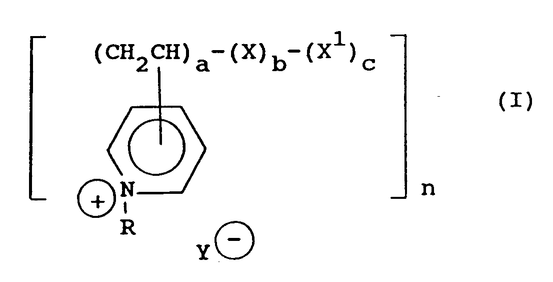
in which,
- R is: C₆₋₂₀ alkyl, hydroxyC₁₋₂₀alkyl or C₁₋₄alkoxyC₁₋₂₀ alkyl;
- Y⁻ is: a counter ion;
- a, b and c: are numbers which indicate the relative percentages (w/w) of the units present in said polymer,
(a) being from 30 to 99 percent (w/w) and
(b) being from 1.0 to 8 percent (w/w);
- n is: a number indicating the degree of polymerisation in said polymer;
- X is: divinylbenzene, or an alkylene glycol bis methacrylate cross-linking unit of structure (i) 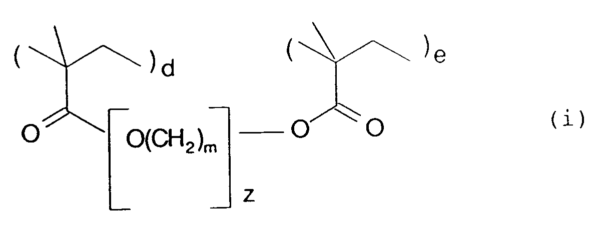 in which m is 2 to 6, z is 1 to 4 and (d) and (e) together comprise from 1.0 to 8 percent (w/w) of said polymer, and
- X¹ is: styrene, an alkyl methacrylate comonomer of structure (ii) or an alkyl styrene comonomer of structure (iii) 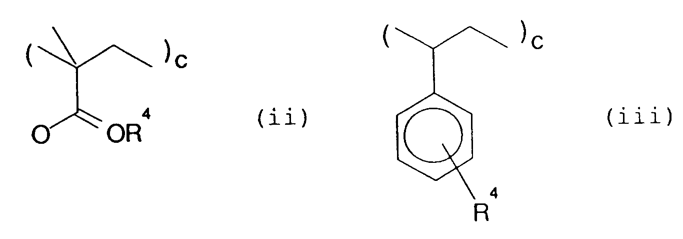 in which R⁴ is C₁₋₂₀alkyl and (c) is as described for structure (I).

Suitably the pyridine ring is attached to the polymer backbone via the 2-, 3- or 4- position of the ring. Preferably the ring is attached via the 3- or 4- positions; most preferably via the 3-position.

Suitably, R is C₆₋₂₀alkyl, hydroxyC₁₋₂₀alkyl or C₁₋₄alkoxyC₁₋₂₀alkyl; more suitably R is a C₆₋₂₀alkyl group; preferably a C₈₋₂₀alkyl group; most preferably a C₈₋₁₆alkyl group.

Preferably R⁴ is C₁₋₆alkyl.

Suitably (a) is from 30 to 99% percent (w/w); preferably from 50 to 99% percent (w/w).

Suitably (b) is from 1 to 8 percent (w/w); preferably (b) is from 1 to 5 percent (w/w), most preferably, 2% percent (w/w).

Suitably (c) is the remaining percent (w/w) present in said polymer. The comonomer unit (c) can be present or absent from the polymer. Preferably (c) is from 0% to 50% percent (w/w); most preferably, from 0% to 25% percent (w/w).

Suitably Y⁻ is a physiologically acceptable counter ion such as a halide, sulphate, phosphate, bicarbonate, carbonate, formate, acetate, sulphonate, propionate, malonate, succinate, malate, tartrate, citrate, maleate, fumarate, ascorbate, glucuronate or the anion of an amino acid such as aspartic or glutamic acid. Preferably Y⁻ is a phosphate, sulphate or halide ion; most preferably a halide ion.

n is a number indicating the degree of polymerisation of the polymer. Owing to the three dimensional cross-linkage precise figures cannot be given for n, but in any case will be greater than 1,000.

The polymers of the present invention are also characterised by their total exchange capacity i.e. the theoretical maximum capacity of the resin if each counter ion were to be exchanged with bile acid. In this specification the total exchange capacity is defined in terms of the number of milliequivalents of counter ion per gram of dry weight of polymer.

Suitable total exchange capacities are in the range of, for example where the counter ion Y⁻ is chlorine, from 1.5 to 6.5 meq Cl⁻ per gram of resin. Preferred within this range are resins having a total exchange capacity of between 2 and 4 meq Cl⁻/gram of resin.

It is to be noted that the term 'bile acid' when used herein shall be taken to include bile acids, bile salts and conjugates thereof.

The polymers of the present invention can be prepared by processes analogous to those known in the art. The present invention therefore provides, in a further aspect, a process for preparing the polymers of structure (I) which comprises reaction of a polymer of structure (II) 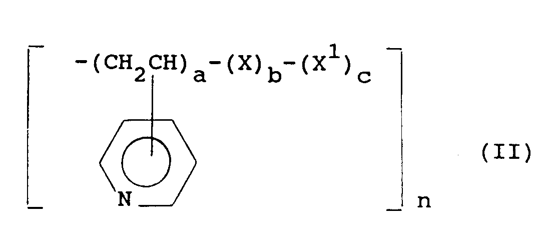
in which a, b, c, X, X¹ and n are as described for structure (I) with a compound of structure RL in which L is a leaving group and R is as described for structure (I).

The reaction between a polymer of structure (II) and a compound RL can be carried out in a suitable solvent at a temperature of between ambient and the reflux temperature of the solvent used. Suitable solvents include for example, a C₁₋₄alkanol, nitromethane, dimethylformamide, tetrahydrofuran or sulpholane. Preferably the reaction is carried out in dimethylformamide or sulpholane at a temperature of between about 60° and 80° for a period of up to 24 hours or until the reaction is complete.

Suitable leaving groups L will be apparent to those skilled in the art and include for example, halides and aryl and alkyl sulphonic acids.

Suitable C₁₋₂₀alkyl halides include the alkyl chlorides bromides and iodides, in particular C₁₋₂₀alkyl iodides.

The intermediate polymers of structure (II) are available commercially or can be prepared from readily available materials by methods known to those skilled in the art. For example polymers of structure (II) in which X is divinylbenzene, and X¹ is styrene can be prepared by reaction of 4-vinylpyridine, styrene and divinyl benzene in an aqueous suspension comprising polyvinyl alcohol in the presence of an initiator at elevated temperature. Suitable initiators will be apparent to those skilled in the art and include, in particular azobisisobutyronitrile.

The polymers of structure (I) have been found to bind bile acids in in vitro models. As indicated earlier it is recognised that removal of bile acids from the intestinal tract lowers serum cholesterol levels and also has a beneficial effect on protecting against atherosclerosis and its dependent clinical conditions. The present invention therefore provides in a further aspect, polymers of structure (I) for use in therapy, in particular for the lowering of serum cholesterol levels in mammals, including humans. In addition the polymers of structure (I) are expected to be of use in protecting against atherosclerosis and its sequelae, and for example, in the treatment of pruritus and diarrhoea.

When used in therapy the polymers of structure (I) are in general administered in a pharmaceutical composition.

In a still further aspect of the present invention there is therefore provided a pharmaceutical composition comprising a polymer of structure (I) in association with a pharmaceutically acceptable carrier.

The compositions of the present invention can be prepared by techniques well known to those skilled in the art of pharmacy and include all those known for the formulation of polymers for human use.

The polymers are preferably administered as formulations in admixture with one or more conventional pharmaceutical excipients which are physically and chemically compatible with the polymer, which are non-toxic, are without deleterious side-effects but which confer appropriate properties on the dosage form.

In general, for liquid formulations aqueous based pharmaceutically acceptable carriers such as water itself or aqueous dilute ethanol, propylene glycol, polyethylene glycol or glycerol or sorbitol solutions are preferred. Such formulations can also include preservatives and flavouring and sweetening agents such as sucrose, fructose, invert sugar, cocoa, citric acid, ascorbic acid, fruit juices etc. In general, digestible oil or fat based carriers should be avoided or minimised as they contribute to the condition sought to be alleviated by use of the polymers. They are also subject to absorption by the polymers during prolonged contact, thus reducing the capacity of the polymer to absorb bile acids after administration.

The polymers can also be prepared as concentrates, for dilution prior to administration, and as formulations suitable for direct oral administration. They can be administered orally ad libitum, on a relatively continuous basis for example by dispersing the polymer in water, drinks or food, for example, in a granule presentation suitable for admixture with water or other drink to provide a palatable drinking suspension.

Preferably, the polymers are administered in tablet form or in gelatin capsules containing solid particulate polymer or a non-aqueous suspension of solid polymer containing a suitable suspending agent. Suitable excipients for such formulations include, for example, for tablets and capsules, lactose, microcrystalline cellulose, magnesium stearate, povidone, sodium starch glycollate and starches; and for suspensions in capsules, polyethylene glycol, propylene glycol and colloidal silicon dioxide. If desired these dosage forms in addition optionally comprise suitable flavouring agents. Alternatively, a chewable tablet or granule presentation incorporating suitable flavouring and similar agents may be used.

Preferably the polymer is administered in unit dosage form, each dosage unit containing preferably from 0.5 g to 1.5 g of polymer.

The daily dosage regimen for an adult patient may be, for example, a total daily oral dose of between 1 and 10 g, preferably 1-5 g, the compound being administered 1 to 4 times a day. Suitably the compound is administered for a period of continuous therapy of one month or more sufficient to achieve the required reduction in serum cholesterol levels.

In addition the polymers of the present invention can be co-administered (together or sequentially) with further active ingredients such as HMGCoA reductase inhibitors and other hypocholesterolaemic agents, and other drugs for the treatment of cardiovascular diseases.

The following biological data and examples indicate the properties and preparation of the polymers of the present invention. Temperatures are recorded in degrees celsius. The exchange capacity of the pyridinium copolymers was determined by elemental analysis and/or potentiometric titration of chloride ion. Figures quoted are expressed as milli equivalents of exchangeable chloride ion per gram of dry resin weight. All percentage figures given are (w/w) figures and are assumed from the initial ratio of monomers used.

### Preparation 1

4-Vinylpyridine (25.0 g), styrene (23.2 g), divinylbenzene (1.8 g, purity 55%) and azobisisobutyronitrile (AIBN) (0.5 g) were mixed to give a homogeneous solution and added to a solution of poly(vinyl alcohol) (m.w. 125,000) (1 g) in distilled water (500 ml) stirred at 80° under an atmosphere of nitrogen. Stirring was continued for 7 hours and then the mixture was poured into distilled water. The resin was washed by decantation with cold and hot water, filtered, and washed with acetone, methanol, methanol/ether and ether, dried to give a 2% cross-linked polymer containing 50% vinylpyridine. This polymer was then sieved and the 53-106µ fraction (16.42 g) was used in the further reactions.

### Preparation 2

In a similar manner starting from 4-vinylpyridine (77.8 g), divinylbenzene (2.88 g, purity 55%), AIBN (0.8 g), poly(vinyl alcohol) (m.w. 125,000) (1 g) and distilled water (500 ml) gave (54.15 g) of a 2% cross-linked polymer containing 96% 4-vinylpyridine.

### Preparation 3

In a similar manner starting from 4-vinylpyridine (60.0 g), styrene (17.12 g), divinylbenzene (2.88 g, purity 55%) AIBN (0.8 g), poly(vinyl alcohol) (m.w. 125,000) (1 g) and distilled water (500 ml) gave (42.48 g) of a 2% cross-linked polymer containing 75% 4-vinylpyridine.

### Preparation 4

In a similar manner starting from 4-vinylpyridine (24.0 g), styrene (53.12 g), divinylbenzene (2.88 g, purity 55%) AIBN (0.8 g), poly(vinyl alcohol) (m.w. 125,000) (1 g) and distilled water (500 ml) gave (37.2 g) of a 2% cross-linked polymer containing 30% 4-vinylpyridine.

### Preparation 5

In a similar manner starting from 4-vinylpyridine (7.25 g), octylstyrene (7.2 g), divinylbenzene (0.55 g, purity 55%) AIBN (0.2 g), poly(vinyl alcohol) (m.w. 125,000) (0.5 g) and distilled water (200 ml) gave (6.63 g) of a 2% cross-linked polymer containing 50%, 4-vinylpyridine.

Octylstyrene was prepared using the following procedure:
(a) Aluminium chloride (84.3 g) was suspended in dry dichloromethane (250 ml), cooled to 10° and acetyl chloride (49.6 g) added over 5 minutes at 10-20°. The mixture was stirred at room temperature for 30 minutes then octylbenzene (100.3 g) was added over 30 minutes to the resulting solution with slight cooling to keep the temperature at 20-22°. After the addition the mixture was stirred at room temperature for 3.5 hours then poured onto ice (1 kg). The phases were separated and the dichloromethane solution was washed with water, dilute sodium hydroxide and water, and dried over magnesium sulphate. Evaporation of the solvent gave 4-acetyl-1-octylbenzene as an oil (117.3 g, 96%).
(b) 4-Acetyl-1-octylbenzene (117.0 g) was dissolved in ethanol (800 ml), cooled to 10° and sodium borohydride (38.2 g) was added in portions over 5 minutes. The mixture was stirred at room temperature for 20 hours. The solvent was evaporated and the residue was partitioned between water and diethyl ether. The diethyl ether solution was washed with water, dilute hydrochloric acid and water, dried over magnesium sulphate and evaporated to give 4-(1-hydroxyethyl)-1-octylbenzene as a colourless oil which solidified on cooling (116.3 g, 98%).
(c) 4-(1-Hydroxyethyl)-1-octylstyrene (113.7 g) was heated at 200° under vacuum (10mmHg) for 0.5 hour. The resulting oil was partitioned between water and diethyl ether and the diethyl ether solution was washed with water, dilute sodium hydroxide and water, dried over magnesium sulphate and evaporated to an oil. This oil was distilled to give 4-octylstyrene as a colourless oil (48.2 g, 46%), b.p. 106-8°/0.03mmHg.

### Preparation 6

In a similar manner starting from 4-vinylpyridine (25.0 g), styrene (24.1 g), divinylbenzene (0.90 g, purity 55%) AIBN (0.5 g), poly(vinyl alcohol) (m.w. 125,000) (1 g) and distilled water (500 ml) gave (17.34 g) of a 1% cross-linked polymer containing 50% 4-vinylpyridine.

### Preparation 7

In a similar manner starting from 4-vinylpyridine (72.8 g), divinylbenzene (7.2 g, purity 55%) AIBN (0.8 g), poly(vinyl alcohol) (m.w. 125,000) (1 g) and distilled water (500 ml) gave (51.56 g) of a 5% cross-linked polymer containing 91% 4-vinylpyridine.

### Preparation 8

In a similar manner starting from 4-vinylpyridine (40.0 g), styrene (32.8 g), divinylbenzene (7.23 g, purity 55%) AIBN (0.8 g), poly(vinyl alcohol) (m.w. 125,000) (1 g) and distilled water (500 ml) gave (44.45 g) of a 5% cross- linked polymer containing 50% 4-vinylpyridine.

### Preparation 9

In a similar manner starting from 4-vinylpyridine (40 g), ethyl methacrylate (38.2 g) ethylene glycol bismethacrylate (1.6 g) AIBN (0.7 g), poly(vinyl alcohol), (m.w. 125,000) (1 g) and distilled water (500 ml) gave (26.44 g) of a 2% cross-linked copolymer containing 50% 4-vinylpyridine.

### Preparation 10

In a similar manner starting from 4-vinylpyridine (25.0 g), ethyl methacrylate (23.2 g), divinylbenzene (1.8 g, purity 55%) AIBN (0.8 g), poly(vinyl alcohol) (m.w. 125,000) (1 g) and distilled water (500 ml) gave (42.48 g) of a 2% cross-linked copolymer containing 50% 4-vinylpyridine.

### Preparation 11

In a similar manner starting from 4-vinylpyridine (25.0 g), dodecyl methacrylate (23.2 g), divinylbenzene (1.8 g, purity 55%), AIBN (0.8 g), poly(vinyl alcohol) (m.w. 125,000) (1 g) and distilled water (500 ml) gave (20.74 g) of a 2% cross-linked copolymer containing 50% 4-vinylpyridine.

### Preparation 12

In a similar manner starting from 4-vinylpyridine (40 g), hexyl methacrylate (38.4 g) ethylene glycol bismethacrylate (1.6 g) AIBN (0.8 g), poly(vinyl alcohol) (m.w. 125,000) (1 g) and distilled water (500 ml) gave (53.95 g) of a 2% cross-linked copolymer containing 50% 4-vinylpyridine.

### Preparation 13

In a similar manner starting from 4-vinylpyridine (40.0 g), hexyl methacrylate (37.1 g), divinylbenzene (2.88 g, purity 55%) AIBN (0.8 g), poly(vinyl alcohol) (m.w. 125,000) (1 g) and distilled water (500 ml) gave (52.76 g) of a 2% cross-linked copolymer containing 50% 4-vinylpyridine.

### Preparation 14

In a similar manner starting from 4-vinylpyridine (40 g), dodecyl methacrylate (38.4 g) ethylene glycol bismethacrylate (1.6 g) AIBN (0.8 g), poly(vinyl alcohol) (m.w. 125,000) (1 g) and distilled water (500 ml) gave (33.70 g) of a 2% cross-linked copolymner containing 50% 4-vinylpyridine.

### Preparation 15

In a similar manner starting from 3-vinylpyridine (15.0 g), styrene (13.9 g), divinylbenzene (0.9 g, purity 55%) AIBN (0.3 g), poly(vinyl alcohol) (m.w. 125,000) (0.5 g) and distilled water (200 ml) gave (14.42 g) of a 2% cross-linked copolymer containing 50% 4-vinylpyridine.

### Preparation 16

In a similar manner starting from 4-vinylpyridine (60.0 g), styrene (18.6 g), divinylbenzene (1.44 g, purity 55%), AIBN (0.8 g), poly(vinyl alcohol) (m.w. 125,000) (1 g) and distilled water (500 ml) gave (39.53 g) of a 1% cross-linked polymer containing 75% 4-vinylpyridine.

### Preparation 17

In a similar manner starting from 3-vinylpyridine (19.28 g), divinylbenzene (0.72 g, purity 55%) AIBN (0.3 g), poly(vinyl alcohol) (m.w. 125,000) (0.5 g) and distilled water (200 ml) gave (9.10 g) of a 2% cross-linked copolymer containing 96% 3-vinylpyridine.

### Example 1

A suspension of the polymer from preparation 1 (3.0 g) in dimethylformamide (60 ml) and octyl iodide (20 ml) was stirred at 80° for 72 hours. The mixture was filtered and the polymer was washed with dimethylformamide, methanol, methanol/dilute hydrochloric acid, methanol, methanol/ether, ether and dried to give 2% cross-linked 1-octyl-4-vinylpyridinium chloride styrene divinylbenzene copolymer (5.19 g) (exchange capacity = 2.59 meq Cl⁻/g).

### Example 2

A suspension of the polymer from preparation 1 (2.08 g) in sulfolane (40 ml) and dodecyl iodide (20 g) was stirred at 80° for 18 hours. The mixture was filtered and the polymer was washed with, methanol, acetone, methanol/dilute hydrochloric acid, methanol, methanol/ether, ether and dried to give 2% cross-linked 1-dodecyl-4-vinylpyridinium chloride styrene divinylbenzene copolymer (3.82 g) (exchange capacity = 2.41 meq Cl⁻/g).

### Example 3

A suspension of the polymer from preparation 1 (3.5 g) in sulfolane (40 ml) and hexadecyl iodide (25 g) was stirred at 90° for 72 hours. The mixture was filtered and the polymer was washed with, methanol, acetone, methanol/dilute hydrochloric acid, methanol, methanol/ether, ether and dried to give 2% cross-linked 1-hexadecyl-4-vinylpyridinium chloride styrene divinylbenzene copolymer (7.20 g) (exchange capacity = 2.1 meq Cl⁻/g).

### Example 4

A suspension of the polymer from preparation 2 (2.00 g) in sulfolane (60 ml) and octyl iodide (20 ml) was stirred at 80° for 18 hours. The mixture was filtered and the polymer was washed with, methanol, methanol/dilute hydrochloric acid, methanol, methanol/ether, ether and dried to give 2% cross-linked 1-octyl-4-vinylpyridinium chloride divinylbenzene copolymer (3.81 g) (exchange capacity = 3.89 meq Cl⁻/g).

### Example 5

A suspension of the polymer from preparation 2 (4.00 g) in sulfolane (75 ml) and dodecyl iodide (25 g) was stirred at 90° for 60 hours. The mixture was filtered and the polymer was washed with, methanol, methanol/dilute hydrochloric acid, methanol, methanol/ether, ether and dried to give 2% cross-linked 1-dodecyl-4-vinylpyridinium chloride divinylbenzene copolymer (11.32 g) (exchange capacity = 3.23 meq Cl⁻/g).

### Example 6

A suspension of the polymer from preparation 2 (4.00 g) in sulfolane (75 ml) and hexadecyl iodide (25 g) was stirred at 90° for 18 hours. The mixture was filtered and the polymer was washed with, acetone, methanol, methanol/dilute hydrochloric acid, methanol, methanol/ether, ether and dried to give 2% cross-linked 1-hexadecyl-4-vinylpyridinium chloride divinylbenzene copolymer (13.28 g) (exchange capacity = 2.77 meq Cl⁻/g).

### Example 7

A suspension of the polymer from preparation 3 (4.00 g) in sulfolane (80 ml) and octyl iodide (25 ml) was stirred at 80° for 56 hours. The mixture was filtered and the polymer was washed with, acetone, methanol, methanol/dilute hydrochloric acid, methanol, methanol/ether, ether and dried to give 2% cross-linked 1-octyl-4-vinylpyridinium chloride styrene divinylbenzene copolymer (7.92 g) (exchange capacity = 3.46 meq Cl⁻/g).

### Example 8

A suspension of the polymer from preparation 3 (4.00 g) in sulfolane (60 ml) and dodecyl iodide (30 g) was stirred at 80° for 56 hours. The mixture was filtered and the polymer was washed with, acetone, methanol, methanol/dilute hydrochloric acid, methanol, methanol/ether, ether and dried to give 2% cross-linked 1-dodecyl-4-vinylpyridinium chloride divinylbenzene styrene copolymer (9.03 g) (exchange capacity = 2.92 meq Cl⁻/g).

### Example 9

A suspension of the polymer from preparation 8 (6.00 g) in sulfolane (60 ml) and octyl iodide (20 ml) was stirred at 80° for 24 hours. The mixture was filtered and the polymer was washed with, methanol, methanol/dilute hydrochloric acid, methanol, methanol/ether, ether and dried to give 5% cross-linked 1-octyl-4-vinylpyridinium chloride styrene divinylbenzene copolymer (9.75 g) (exchange capacity = 2.73 meq Cl⁻/g).

### Example 10

A suspension of the polymer from preparation 7 (6.00 g) in sulfolane (60 ml) and octyl iodide (20 ml) was stirred at 80° for 24 hours. The mixture was filtered and the polymer was washed with, methanol, methanol/dilute hydrochloric acid, methanol, methanol/ether, ether and dried to give 5% cross-linked 1-octyl-4-vinylpyridinium chloride divinylbenzene copolymer (12.87 g) (exchange capacity = 3.75 meq Cl⁻/g).

### Example 11

A suspension of the polymer from preparation 6 (3.00 g) in sulfolane (50 ml) and octyl iodide (10 ml) was stirred at 80° for 20 hours. The mixture was filtered and the polymer was washed with, methanol, methanol/dilute hydrochloric acid, methanol, methanol/ether, ether and dried to give 1% cross-linked 1-octyl-4-vinylpyridinium chloride styrene divinylbenzene copolymer (4.39 g) (exchange capacity = 2.66 meq Cl⁻/g).

### Example 12

A suspension of the polymer from preparation 4 (6.00 g) in sulfolane (60 ml) and octyl iodide (20 ml) was stirred at 80° for 24 hours. The mixture was filtered and the polymer was washed with, methanol, methanol/dilute hydrochloric acid, methanol, methanol/ether, ether and dried to give 2% cross-linked 1-octyl-4-vinylpyridinium chloride styrene divinylbenzene copolymer (8.06 g) (exchange capacity = 2.11 meq Cl⁻/g).

### Example 14

A suspension of the polymer from preparation 1 (4.00 g) in sulfolane (40 ml) and decyl iodide (20 ml) was stirred at 80° for 24 hours. The mixture was filtered and the polymer was washed with methanol, methanol/dilute hydrochloric acid, methanol, methanol/ether, ether and dried to give 2% cross-linked 1-tetradecyl-4-vinyl- pyridinium chloride styrene divinylbenzene copolymer (7.0 g) (exchange capacity = 2.57 meq Cl⁻/g).

### Example 15

A suspension of the polymer from preparation 9 (3.49 g) in sulfolane (50 ml) and octyl iodide (25 ml) was stirred at 80° for 18 hours. The mixture was filtered and the polymer was washed with methanol, acetone, methanol/ dilute hydrochloric acid, methanol, methanol/ether, ether and dried to give 2% cross-linked 1-octyl-4-vinyl- pyridinium chloride ethyl methacrylate ethylene glycol bismethacrylate copolymer (5.68 g) (exchange capacity = 2.86 meq Cl⁻/g).

### Example 16

A suspension of the polymer from preparation 10 (3.0 g) in sulfolane (50 ml) and octyl iodide (25 ml) was stirred at 80° for 56 hours. The mixture was filtered and the polymer was washed with methanol, acetone, methanol/dilute hydrochloric acid, methanol, methanol/ether, ether and dried to give 2% cross-linked 1-octyl-4-vinylpyridinium chloride ethyl methacrylate divinylbenzene copolymer (4.07 g) (exchange capacity = 2.85 meq Cl⁻/g).

### Example 17

A suspension of the polymer from preparation 1 (2.00 g) in sulfolane (30 ml) and 11-bromoundecanol (4.0 g) was stirred at 80° for 24 hours. The mixture was filtered and the polymer was washed with methanol, methanol/dilute hydrochloric acid, methanol, methanol/ether, ether and dried to give 2% cross-linked 1-(11-hydroxyundecyl)-4-vinylpyridinium chloride styrene divinylbenzene copolymer (3.73 g) (exchange capacity = 2.38 meq Cl⁻/g).

### Example 18

A suspension of the polymer from preparation 9 (3.00 g) in sulfolane (50 ml) and dodecyl iodide (25 ml) was stirred at 90° for 18 hours. The mixture was filtered and the polymer was washed with methanol, methanol/dilute hydrochloric acid, methanol, methanol/ether, ether and dried to give 2% cross-linked 1-dodecyl-4-vinylpyridinium chloride ethyl methacrylate ethylene glycol bismethacrylate copolymer (5.56 g) (exchange capacity = 2.71 meq Cl⁻/g).

### Example 19

A suspension of the polymer from preparation 11 (4.00 g) in sulfolane (50 ml) and octyl iodide (25 ml) was stirred at 90° for 18 hours. The mixture was filtered and the polymer was washed with methanol, methanol/dilute hydrochloric acid, methanol, methanol/ether, ether and dried to give 2% cross-linked 1-octyl-4-vinylpyridinium chloride dodecyl methacrylate divinylbenzene copolymer (6.81 g) (exchange capacity = 3.0 meq Cl⁻/g).

### Example 21

A suspension of the polymer from preparation 6 (4.0 g) in sulfolane (50 ml) and dodecyl iodide (20 ml) was stirred at 80° for 24 hours. The mixture was filtered and the polymer was washed with methanol, acetone, methanol/dilute hydrochloric acid, methanol, methanol/ether, ether and dried to give 1% cross-linked 1-dodecyl-4-vinylpyridinium chloride styrene divinylbenzene copolymer (6.78 g) (exchange capacity = 2.3 meq Cl⁻/g).

### Example 22

A suspension of the polymer from preparation 8 (4.0 g) in sulfolane (50 ml) and dodecyl iodide (20 ml) was stirred at 80° for 24 hours. The mixture was filtered and the polymer was washed with methanol, acetone, methanol/dilute hydrochloric acid, methanol, methanol/ether, ether and dried to give 5% cross-linked 1-dodecyl-4-vinylpyridinium chloride styrene divinylbenzene copolymer (7.27) (exchange capacity = 2.48 meq Cl⁻/g).

### Example 24

A suspension of the polymer from preparation 13 (5.00 g) in sulfolane (50 ml) and dodecyl iodide (25 ml) was stirred at 80° for 24 hours. The mixture was filtered and the polymer was washed with methanol, methanol/dilute hydrochloric acid, methanol, methanol/ether, ether and dried to give 2% cross-linked 1-dodecyl-4-vinylpyridinium chloride hexyl methacrylate divinylbenzene copolymer (7.73 g) (exchange capacity = 2.35 meq Cl⁻/g).

### Example 25

A suspension of the polymer from preparation 12 (5.00 g) in sulfolane (50 ml) and octyl iodide (20 ml) was stirred at 80° for 24 hours. The mixture was filtered and the polymer was washed with methanol, methanol/dilute hydrochloric acid, methanol, methanol/ether, ether and dried to give 2% cross-linked 1-octyl-4-vinylpyridinium chloride hexyl methacrylate ethylene glycol bismethacrylate copolymer (7.30 g) (exchange capacity = 2.71 meq Cl⁻/g).

### Example 26

A suspension of the polymer from preparation 2 (2.50 g) in sulfolane (50 ml) and 11-bromoundecanol (10.0 g) was stirred at 80° for 24 hours. The mixture was filtered and the polymer was washed with methanol, methanol/dilute hydrochloric acid, methanol, methanol/ether, ether and dried to give 2% cross-linked 1-(11-hydroxyundecyl)-4-vinylpyridinium chloride divinylbenzene copolymer (6.67 g) (exchange capacity = 3.19 meq Cl⁻/g).

### Example 27

A suspension of the polymer from preparation 14 (4.00 g) in sulfolane (60 ml) and octyl iodide (20 ml) was stirred at 60° for 72 hours. The mixture was filtered and the polymer was washed with methanol, methanol/dilute hydrochloric acid, methanol, methanol/ether, ether and dried to give 2% cross-linked 1-octyl-4-vinylpyridinium chloride dodecyl methacrylate ethylene glycol bismethacrylate copolymer (5.76 g) (exchange capacity = 2.62 meq Cl⁻/g).

### Example 28

A suspension of the polymer from preparation 3 (4.00 g) in sulfolane (60 ml) and hexadecyl iodide (30 g) was stirred at 80° for 56 hours. The mixture was filtered and the polymer was washed with acetone, methanol, methanol/dilute hydrochloric acid, methanol, methanol/ether, ether and dried to give 2% cross-linked 1-hexadecyl-4-vinylpyridinium chloride styrene divinylbenzene copolymer (8.77 g) (exchange capacity = 2.49 meq Cl⁻/g).

### Example 29

A suspension of the polymer from preparation 15 (4.00 g) in sulfolane (40 ml) and dodecyl iodide (25 ml) was stirred at 80° for 24 hours. The mixture was filtered and the polymer was washed with methanol, methanol/dilute hydrochloric acid, methanol, methanol/ether, ether and dried to give 2% cross-linked 1-dodecyl-3-vinylpyridinium chloride styrene divinylbenzene copolymer (6.90 g) (exchange capacity = 2.30 meq Cl⁻/g).

### Example 30

A suspension of the polymer from preparation 16 (4.00 g) in sulfolane (50 ml) and dodecyl iodide (20 ml) was stirred at 80° for 24 hours. The mixture was filtered and the polymer was washed with methanol, methanol/dilute hydrochloric acid, methanol, methanol/ether, ether and dried to give 1% cross-linked 1-dodecyl-4-vinylpyridinium chloride styrene divinylbenzene copolymer (9.21 g) (exchange capacity = 2.89 meq Cl⁻/g).

### Example 31

A suspension of the polymer from preparation 13 (5.00 g) in sulfolane (50 ml) and octyl iodide (25 ml) was stirred at 80° for 24 hours. The mixture was filtered and the polymer was washed with methanol, methanol/dilute hydrochloric acid, methanol, methanol/ether, ether and dried to give 2% cross-linked 1-octyl-4-vinylpyridinium chloride hexyl methacrylate divinylbenzene copolymer (8.03 g) (exchange capacity = 2.71 meq Cl⁻/g).

### Example 32

A suspension of the polymer from preparation 12 (5.00 g) in sulfolane (50 ml) and dodecyl iodide (25 ml) was stirred at 80° for 24 hours. The mixture was filtered and the polymer was washed with methanol, methanol/dilute hydrochloric acid, methanol, methanol/ether, ether and dried to give 2% cross-linked 1-dodecyl-4-vinylpyridinium chloride hexyl methacrylate ethylene glycol bismethacrylate copolymer (9.1 g) (exchange capacity = 2.35 meq Cl⁻/g).

### Example 33

A suspension of the polymer from preparation 1 (3.00 g) in sulfolane (30 ml) and tetradecyl bromide (25 ml) was stirred at 80° for 24 hours. The mixture was filtered and the polymer was washed with methanol, methanol/dilute hydrochloric acid, methanol, methanol/ether, ether and dried to give 2% cross-linked 1-tetradecyl-4-vinylpyridinium chloride styrene divinylbenzene copolymer (5.85 g) (exchange capacity = 2.20 meq Cl⁻/g).

### Example 34

A suspension of the polymer from preparation 16 (5.00 g) in sulfolane (50 ml) and octyl iodide (25 ml) was stirred at 80° for 24 hours. The mixture was filtered and the polymer was washed with methanol, methanol/dilute hydrochloric acid, methanol, methanol/ether, ether and dried to give 1% cross-linked 1-octyl-4-vinylpyridinium chloride styrene divinylbenzene copolymer (9.30 g) (exchange capacity = 3.48 meq Cl⁻/g).

### Example 35

A suspension of the polymer from preparation 17 (4.00 g) in sulfolane (40 ml) and dodecyl iodide (25 ml) was stirred at 80° for 24 hours. The mixture was filtered and the polymer was washed with methanol, methanol/dilute hydrochloric acid, methanol, methanol/ether, ether and dried to give 2% cross-linked 1-dodecyl-3-vinylpyridinium chloride styrene divinylbenzene copolymer (4.87 g) (exchange capacity = 3.07 meq Cl⁻/g).

### Example 35

A suspension of the polymer from preparation 17 (3.00 g) in sulfolane (30 ml) and octyl iodide (20 ml) was stirred at 80° for 24 hours. The mixture was filtered and the polymer was washed with methanol, methanol/dilute hydrochloric acid, methanol, methanol/ether, ether and dried to give 2% cross-linked 1-octyl-3-vinylpyridinium chloride divinylbenzene copolymer (6.67 g) (exchange capacity = 3.53 meq Cl⁻/g).

### Example A

A chewable tablet composition can be prepared from the following :

| | mg/tablet |
|---|---|
| Compound of Structure (I) | 1250 |
| Silicon dioxide | 15 |
| Microcrystalline cellulose | 280 |
| Sorbitol | 445 |
| Lactose | 450 |
| Sweetener | 5 |
| Peppermint | 30 |
| Magnesium Stearate | 25 |
| | 2̅5̅0̅0̅mg |

### Example B

A food additive composition, for example, a sachet for reconstitution or mixing with food, is prepared by incorporating into a powder formulation compound of Structure (I) (250 mg), sodium carboxymethylcellulose (50 mg), sucrose (2400 mg) and flavours (50 mg).

### Data

### In vitro Dissociation assay

The following assay provides a measure of affinity of the polymers of the invention for the bile acid, glycocholate (GC) based on the amount of GC bound at a subsaturating concentration of 5 mM (t=o) and an estimate of the rate at which this bile acid dissociates into a large volume of buffer. The results are given as initial amounts of GC bound (t=O); amounts remaining bound after 2 minutes in buffer (t=2 min), and as % dissociation i.e. the proportion of bound GC dissociated from the polymer after 2 minutes. The lower the % dissociation the more efficient the polymer can be expected to be in extracting bile acids in vivo.

### ° Method

Test compound (150 mg) was equilibrated with 5 mM sodium glycocholate (30 ml) in Krebs' buffer. The compound was separated by centrifugation and the total bound determined by subtraction of the amount in the supernatant from the total bile acid used. Dissociation was measured by resuspending the compound in Krebs' buffer, stirring and sampling the mixture through a filter at several time points up to 20 minutes. Radioactivity and hence bile acid dissociated was determined in the filtrate.

### ° Results

The following % dissociation figures were obtained

| Examples | % Dissociation (range) |
|---|---|
| 2,4,5,6,7,10,11,14,18,21,22,24,28,29,30 and 32-35 | 2-10 |
| 3,8,9,12,15,16,17,19,25,26,27 and 31 | 11-22 |

## Claims (Claims for the following Contracting State(s): AT, BE, CH, DE, FR, GB, IT, LI, LU, NL, SE)

1. A cross-linked polymer of structure 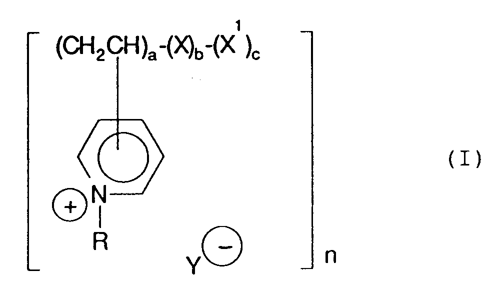 in which,
R is C₆₋₂₀alkyl, hydroxyC₁₋₂₀alkyl or C₁₋₄alkoxyC₁₋₂₀alkyl;
Y⁻ is a counter ion;
a, b and c are numbers which indicate the relative percentages (w/w) of the units present in said polymer,
(a) being from 30 to 99 percent (w/w), and
(b) being from 1.0 to 8 percent (w/w);
n is a number indicating the degree of polymerisation in said polymer;
X is divinylbenzene, or alkylene glycol bis methacrylate cross-linking unit of structure (i) 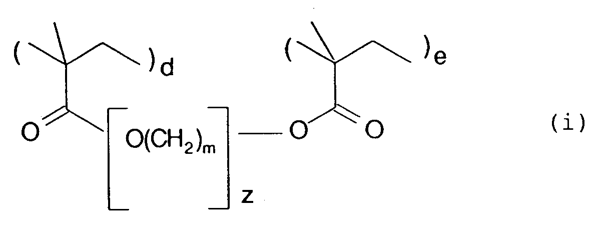 in which m is 2 to 6, z is 1 to 4 and (d) and (e) together comprise from 1.0 to 8 percent (w/w) of said polymer, and
X¹ is styrene, an alkyl methacrylate comonomer of structure (ii) or an alkyl styrene comonomer of structure (iii) 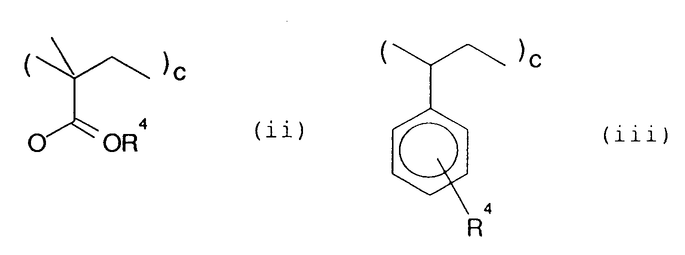 in which R⁴ is C₁₋₂₀alkyl and (c) is as described for structure (I).

2. A polymer according to claim 1 in which R is a C₈₋₁₆ alkyl group.

3. A polymer according to claim 2 in which (b) is from 1 to 5 percent (w/w) of said polymer.

4. A process for the preparation of a polymer according to any one of claims 1 to 3 which comprises reaction of a polymer of structure (II) 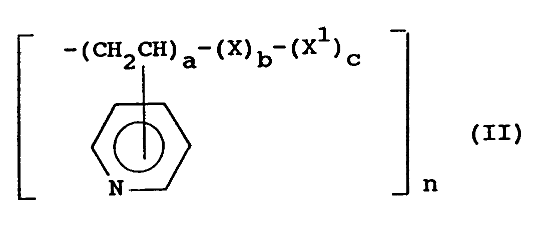 in which a, b, c, X, X¹ and n are as described for structure (I) with a compound of structure RL in which L is a leaving group and R is as described for structure (I).

5. A pharmaceutical composition comprising a polymer of structure (I) as claimed in claim 1 in association with a pharmaceutically acceptable carrier.

6. A polymer according to claim 1 for use as a therapeutic agent.

7. A polymer according to claim 1 for use in the lowering of serum cholesterol levels.

## Claims (Claims for the following Contracting State(s): GR, ES)

1. A process for the preparation of a polymer of structure (I) 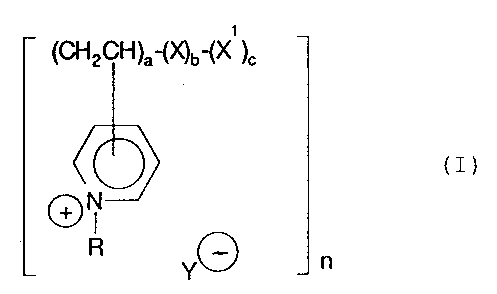 in which,
R is C₆₋₂₀ alkyl, hydroxyC₁₋₂₀alkyl or C₁₋₄alkoxyC₁₋₂₀alkyl;
Y⁻ is a counter ion;
a, b and c are numbers which indicate the relative percentages (w/w) of the units present in said polymer,
(a) being from 30 to 99 percent (w/w) and
(b) being from 1.0 to 8 percent (w/w);
n is a number indicating the degree of polymerisation in said polymer;
X is divinylbenzene, or alkylene glycol bis methacrylate cross-linking unit of structure (i) 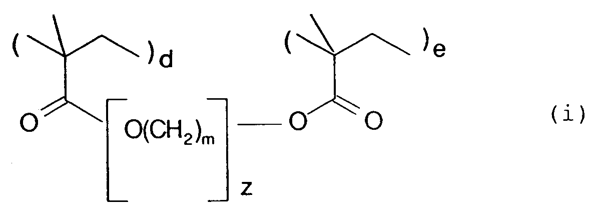 n which m is 2 to 6, z is 1 to 4 and (d) and (e) together comprise from 1.0 to 8 percent (w/w) of said polymer, and
X¹ is styrene, an alkyl methacrylate comonomer of structure (ii) or an alkyl styrene comonomer of structure (iii) 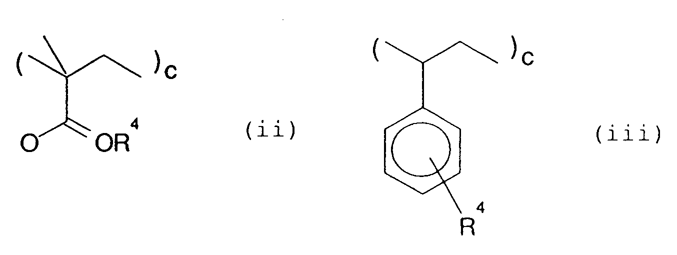 in which R⁴ is C₁₋₂₀alkyl and (c) is as described for structure (I),
which comprises reaction of a compound of structure (II) 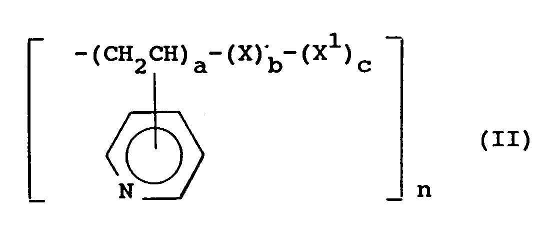 in which a, b, c, X, X¹ and n are as described for structure (I) with a compound of structure RL in which R is as described for structure (I) and L is a leaving group.

2. A process according to claim 1 in which in structure (I) R is a C₈₋₁₆alkyl group.

3. A process according to claim 2 in which (b) is from 1 to 5 percent (w/w) of said polymer.

4. A process according to claim 3, in which L is halogen.

5. A process for preparing a pharmaceutical composition which comprises bringing into association a polymer of structure (I) as described in claim 1 and a pharmaceutically acceptable carrier.

## Patentansprüche (Patentansprüche für folgende(n) Vertragsstaat(en): AT, BE, CH, DE, FR, GB, IT, LI, LU, NL, SE)

1. Vernetztes Polymer der Struktur 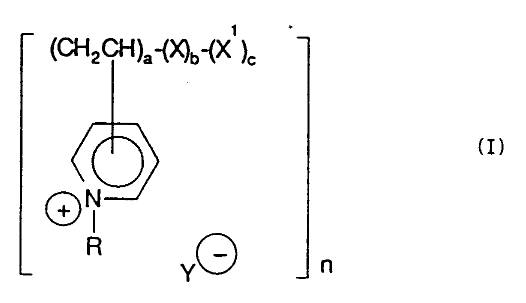 in der
R einen C₆₋₂₀-Alkyl-, Hydroxy(C₁₋₂₀)alkyl- oder C₁₋₄-Alkoxy(C₁₋₂₀)-alkylrest bedeutet;
Y⁻ ein Gegenion ist;
a, b und c Zahlen bedeuten, die die relativen prozentualen Anteile (w/w) der in dem Polymer vorliegenden Einheiten angeben, wobei
(a) 30 bis 99 % (w/w) ist, und
(b) 1,0 bis 8 % (w/w) ist;
n eine Zahl bedeutet, die den Polymerisationsgrad in dem Polymer angibt;
X Divinylbenzol oder eine vernetzende Alkylenglykolbismethacrylat-Einheit der Struktur (i) 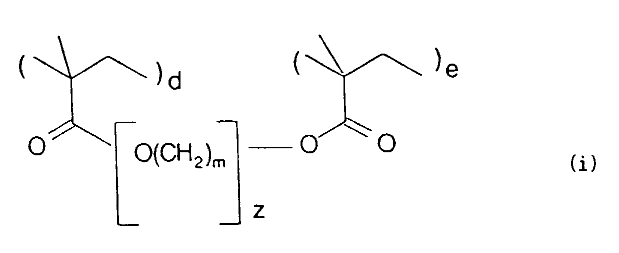 ist, in der m 2 bis 6 ist, z 1 bis 4 ist, und (d) und (e) zusammen 1,0 bis 8 % (w/w) des Polymers umfassen, und
X¹ Styrol, ein Alkylmethacrylatcomonomer der Struktur (ii) oder ein Alkylstyrolcomonomer der Struktur (iii) 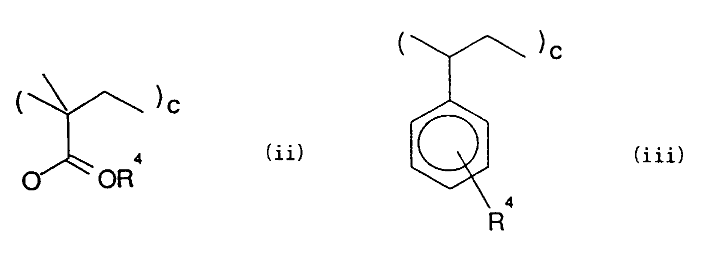 bedeutet, in der R⁴ ein C₁₋₂₀-Alkylrest ist, und (c) wie für Struktur (I) beschrieben ist.

2. Polymer nach Anspruch 1, wobei R ein C₈₋₁₆-Alkylrest ist.

3. Polymer nach Anspruch 2, wobei (b) 1 bis 5 % (w/w) des Polymers ist.

4. Verfahren zur Herstellung eines Polymers nach einem der Ansprüche 1 bis 3, das die Umsetzung eines Polymers der Struktur (II) 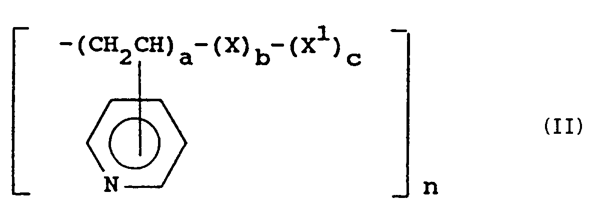 in der a, b, c, X, X¹ und n wie für Struktur (I) beschrieben sind, mit einer Verbindung der Struktur RL umfaßt, in der L eine Abgangsgruppe bedeutet, und R wie für Struktur (I) beschrieben ist.

5. Arzneimittel, umfassend eine Polymer der Struktur (I) nach Anspruch 1 in Verbindung mit einem pharmazeutisch verträglichen Träger.

6. Polymer nach Anspruch 1 zur Verwendung als Arzneimittel.

7. Polymer nach Anspruch 1 zur Verwendung zur Senkung der Cholesterinkonzentrationen im Serum.

## Patentansprüche (Patentansprüche für folgende(n) Vertragsstaat(en): GR, ES)

1. Verfahren zur Herstellung eines Polymers der Struktur (I) 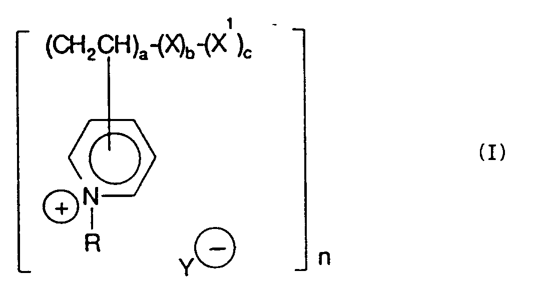 in der
R einen C₆₋₂₀-Alkyl-, Hydroxy(C₁₋₂₀)alkyl- oder C₁₋₄-Alkoxy(C₁₋₂₀)-alkylrest bedeutet;
Y⁻ ein Gegenion ist;
a, b und c Zahlen bedeuten, die die relativen prozentualen Anteile (w/w) der in dem Polymer vorliegenden Einheiten angeben, wobei
(a) 30 bis 99 % (w/w) ist, und
(b) 1,0 bis 8 % (w/w) ist;
n eine Zahl bedeutet, die den Polymerisationsgrad in dem Polymer angibt;
X Divinylbenzol oder eine vernetzende Alkylenglykolbismethacrylat-Einheit der Struktur (i) 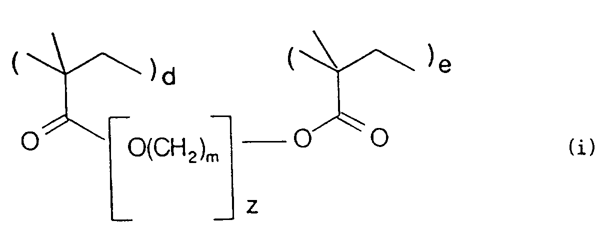 ist, in der m 2 bis 6 ist, z 1 bis 4 ist, und (d) und (e) zusammen 1,0 bis 8 % (w/w) des Polymers umfassen, und
X¹ Styrol, ein Alkylmethacrylatcomonomer der Struktur (ii) oder ein Alkylstyrolcomonomer der Struktur (iii) 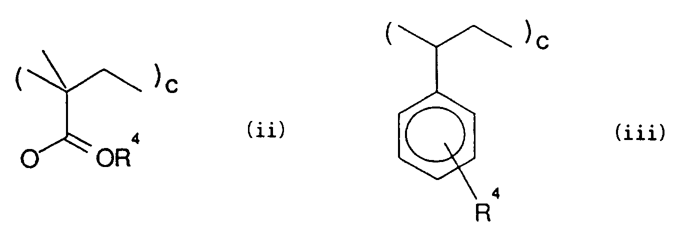 bedeutet, in der R⁴ ein C₁₋₂₀-Alkylrest ist, und (c) wie für Struktur (I) beschrieben ist,
das die Umsetzung einer Verbindung der Struktur (II) 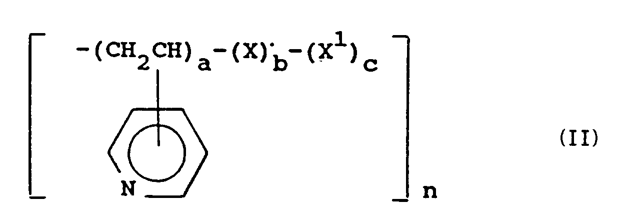 in der a, b, c, X, X¹ und n wie für Struktur (I) beschrieben sind, mit einer Verbindung der Struktur RL umfaßt, in der R wie für Struktur (I) beschrieben ist, und L eine Abgangsgruppe bedeutet.

2. Verfahren nach Anspruch 1, wobei in R Struktur (I) ein C₈₋₁₆-Alkylrest ist.

3. Verfahren nach Anspruch 2, wobei (b) 1 bis 5 % (w/w) des Polymers ist.

4. Verfahren nach Anspruch 3, wobei L ein Halogenatom ist.

5. Verfahren zur Herstellung eines Arzneimittels, das das Zusammenbringen eines Polymers der Struktur (I), wie in Anspruch 1 beschrieben, und eines pharmazeutisch verträglichen Trägers umfaßt.

## Revendications (Revendications pour l'(les) Etat(s) contractant(s) suivant(s): AT, BE, CH, DE, FR, GB, IT, LI, LU, NL, SE)

1. Polymère réticulé de structure (I): 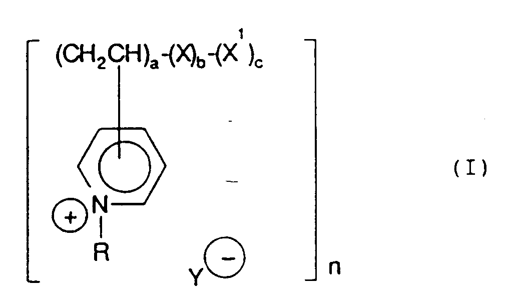 dans laquelle:
- R représente un groupe alkyle C₆₋₂₀, un groupe hydroxyalkyle C₁₋₂₀ ou alcoxy C₁₋₄ alkyle C₁₋₂₀;
- Y⁻ est un contre-ion;
- a, b et c sont les nombres indiquant les pourcentages m/m relatifs des unités présentes dans ledit polymère, (a) étant compris entre 30 et 99 % (m/m) et (b) étant compris entre 1,0 et 8 % (m/m);
- n est un nombre indiquant le degré de polymérisation dudit polymère;
- X représente l'unité de réticulation divinylbenzène ou bis méthacrylate d'alkylèneglycol, de structure (i) 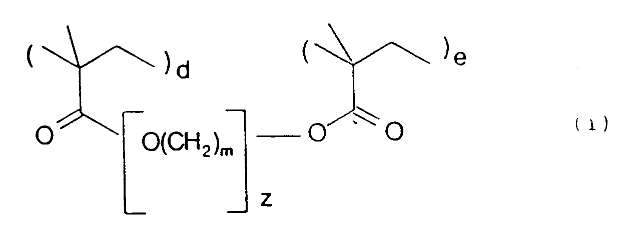 dans laquelle m est compris entre 2 et 6, z entre 1 et 4, et (d) et (e) ensemble représentent 1,0 à 8 % (m/m) dudit polymère, et
- X¹ représente le styrène, un comonomère méthacrylate d'alkyle de structure (ii) ou un comonomère alkylstyrène de structure (iii) 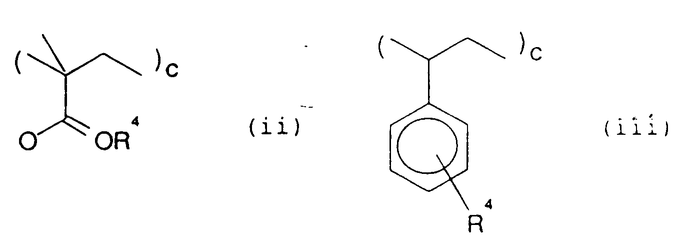 dans laquelle R⁴ est un groupe alkyle C₁₋₂₀ et (c) est tel que décrit pour la structure (I).

2. Polymère suivant la revendication 1, dans lequel R est un groupe alkyle C₈₋₁₆.

3. Polymère suivant la revendication 2, dans lequel (b) représente 1 à 5 % (m/m) dudit polymère.

4. Procédé de préparation d'un polymère suivant l'une quelconque des revendications 1 à 3, qui comprend la réaction d'un polymère de structure (II) 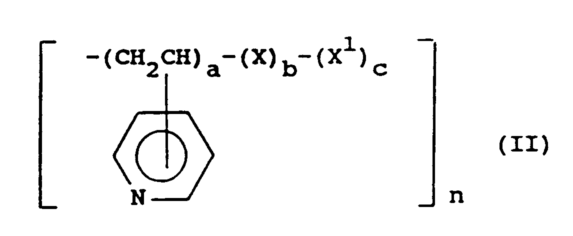 dans laquelle a, b, c, X, X¹ et n sont tels que décrits pour la structure (I), avec un composé de structure RL dans laquelle L est un groupe partant et R est tel que décrit pour la structure (I).

5. Composition pharmaceutique comprenant un polymère de structure (I) suivant la revendication 1, en association avec un véhicule pharmaceutiquement acceptable.

6. Polymère suivant la revendication 1, pour l'utilisation comme agent thérapeutique.

7. Polymère suivant la revendication 1, pour l'utilisation dans la réduction des faux de cholestérol dans le sérum.

## Revendications (Revendications pour l'(les) Etat(s) contractant(s) suivant(s): GR, ES)

1. Procédé de préparation d'un polymère réticulé de structure(I): 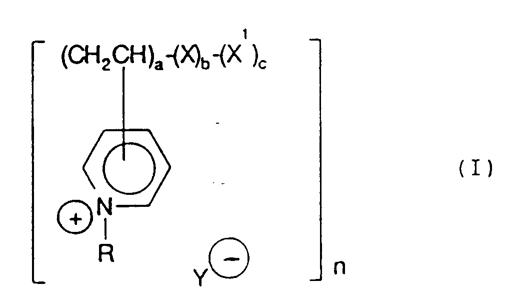 dans laquelle:
- R représente un groupe alkyle C₆₋₂₀, un groupe hydroxyalkyle C₁₋₂₀ ou alcoxy C₁₋₄ alkyle C₁₋₂₀;
- Y⁻ est un contre-ion;
- a, b et c sont les nombres indiquant les pourcentages relatifs m/m des unités présentes dans ledit polymère, (a) étant compris entre 30 et 99 % (m/m) et (b) étant compris entre 1,0 et 8 % (m/m);
- n est un nombre indiquant le degré de polymérisation dudit polymère;
- X représente l'unité de réticulation divinylbenzène ou bis méthacrylate d'alkylèneglycol, de structure (i) 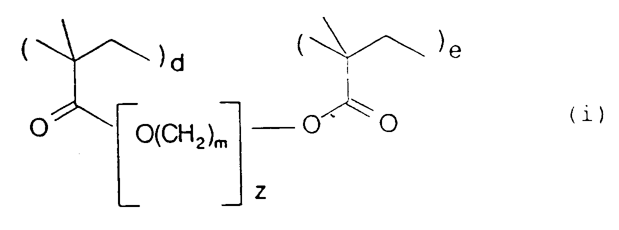 dans laquelle m est compris entre 2 et 6, z entre 1 et 4, et (d) et (e) ensemble représentent 1,0 à 8 % (m/m) dudit polymère, et
- X¹ représente le styrène, un comonomère méthacrylate d'alkyle de structure (ii) ou un comonomère alkylstyrène de structure (iii) 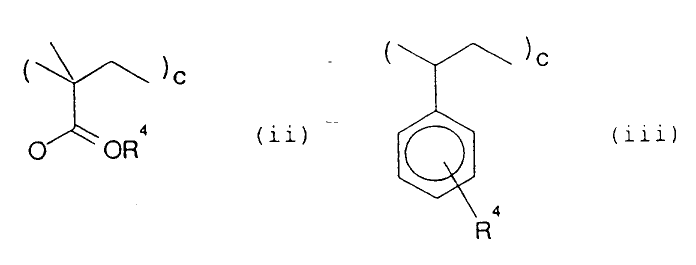 dans laquelle R⁴ est un groupe alkyle C₁₋₂₀ et (c) est tel que décrit pour la structure (I), et qui comprend la réaction d'un composé de structure (II) 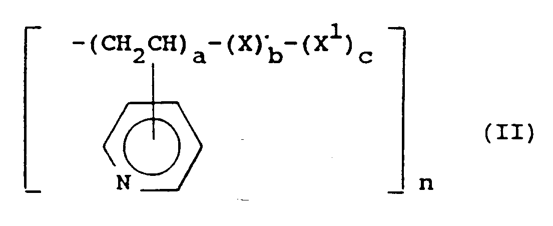 dans laquelle a, b, c, X, X¹ et n sont tels que décrits pour la structure (I), avec un composé de structure RL dans laquelle R est tel que décrit pour la structure (I) et L est un groupe partant.

2. Procédé suivant la revendication 1, dans lequel, pour la structure (I), R est un groupe alkyle C₈₋₁₆.

3. Procédé suivant la revendication 2, dans lequel (b) représente 1 à 5 % (m/m) dudit polymère.

4. Procédé suivant la revendication 3, dans lequel L est un halogène.

5. Procédé de préparation d'une composition pharmaceutique comprenant la mise en association d'un polymère de structure (I) suivant la revendication 1, avec un véhicule pharmaceutiquement acceptable.
